# EUROPEAN PATENT APPLICATION

(11) **EP 4 082 471 A1**
(43) Date of publication of application: **02.11.2022**
(21) Application number: 21170648.6
(22) Date of filing: 27.04.2021
(51) Int. Cl.: A61B 42/20, A41D 13/08, A61B 42/40, A61B 42/50

(54) **FINGER COVER DISPENSER**

(71) Applicant: Paprtect AB, 554 75 Jönköping (SE)
(72) Inventor: Thorwalls, Ola, 55466 Jönköping (SE); Elwing, Erik, 55466 Jönköping (SE)
(74) Representative: AWA Sweden AB

(57) **Abstract**

The present invention relates to a finger cover dispenser (1, 2) comprising a housing (10, 20), and a feeder mechanism (30, 40) configured to feed finger covers (5, 6) to the housing. The housing comprises an application opening (51) which is configured to, when in use, enable a user to apply the finger cover to a finger by putting the finger (9) together with a finger cover received from the feeder mechanism into the application opening. The invention further relates to a method for applying a finger cover (5) to a user's finger (9).

## Description

### Technical Field

The present disclosure relates to a dispenser, and especially a finger cover dispenser for application of a finger cover to a finger of a user.

### Background

In public areas, such as restaurants, airports, hotels etc., it is more and more common for a user, visitor or customer to perform operations such as ordering and check-ins by themselves via a touch screen. Such public touch screen may be used by numerous different persons every hour, and thereby providing a high risk surface to contain bacteria and colony-forming units. In order to minimize spread of infections between humans via such touch screen, cleaning of the touch surface would need to be performed after each use, which is normally not practically possible.

An alternative to cleaning of the surface after each use may be to provide disposable articles for the user to cover the finger or hand when using the touch screen. Example of such articles may be found in e.g. US2013025016 and DE102004006059. However, such available finger covers are either complicated to attach to the finger, and/or are bulky and cost-inefficient.

Consequently, there is a need for a way to enable a user of a public touch screen to quick and easy apply, and dispose, a finger cover, which is easy and cost-efficient for an operator to provide.

### Summary

It is an object of the present invention to provide an improved solution that alleviates the mentioned drawbacks with present devices. Furthermore, it is an object to provide device that enable a user to apply a finger cover easily and securely.

The invention is defined by the appended independent claims, with embodiments being set forth in the appended dependent claims, in the following description and in the drawings.

According to a first aspect of the invention, a finger cover dispenser is provided. The finger cover dispenser comprises a housing, and a feeder mechanism configured to feed finger covers to the housing. The housing comprises an application opening which is configured to, when in use, enable a user to apply the finger cover to a finger by putting the finger together with a finger cover received from the feeder mechanism into the application opening.

The finger cover dispenser may enable a user to access a finger cover supplied by the feeder mechanism and put the finger together with the finger cover into the application opening. The application opening may be configured to receive a finger of a user and, when the finger is put into the application opening, form the finger cover to enclose the finger. The application opening may be a circular opening. The application opening may further be a cylindrical hole into which a user, when in use, may put a finger together with a finger cover. When referring to the finger of a user it may be meant a portion of the finger or the tip of the finger.

The finger covers fed by the feeder mechanism may be flat shaped. The finger cover dispenser may comprise a plurality of finger covers configured to be fed to the housing by the feeder mechanism. The finger covers may be disposable finger covers, and may be made of e.g. a paper material. The material of the finger covers may be selected to facilitate the forming around a user's fingertip, and also the preservation of such form after application to the finger. Such properties may be provided by the thickness, material stiffness, surface properties and/or density of the material. The finger covers may in one embodiment be provided with a sticky coating on one side thereof to facilitate the application of a finger cover to a user's finger. The sticky coating may be selected to have a stickiness facilitating the application of a finger cover to a user's finger, and at the same time not too sticky to prevent separation of finger covers being in contact in the feeder mechanism. A relatively low stickiness may be sufficient to achieve the function of facilitating the forming of the finger cover around a finger.

The feeder mechanism may be configured to feed finger covers in a consecutive manner, making one finger cover at the time accessible to a user at the housing of the dispenser. The feeder mechanism may further be configured such that present finger cover is accessible to the user at a first position, and a removal of the present finger cover from the first position provides a subsequent finger cover to be fed to the first position. When a user is moving the present finger cover from the first position, the present finger cover may be moved to an application position adjacent to the application opening. The application position may be a position at which the finger cover may be pushed into the application opening. The application position may be a position different from the first position. When the present finger cover is at the application position, a subsequent finger cover may have been fed to the first position. When at the application position, the present finger cover may be pushed into the application opening by a user's finger, thereby being formed to enclose the finger. Alternatively, the first position and the application position may be at the same location, providing that the present finger cover may be moved from the first position when the present finger cover is pushed into the application opening. The feeder mechanism may then be configured to feed a subsequent finger cover to the first position in response to the present finger cover have been pushed into the application opening.

The feeder mechanism may be configured to feed a subsequent finger cover automatically following the application of a finger cover to a user's finger. Such feeding may be provided by a mechanical mechanism, or an electrically driven mechanism.

In one embodiment, the application opening may be a circular opening having a diameter of between 10-30 mm, preferably between 12-20 mm. The application opening may be substantially cross-sectionally finger shaped. When a user pushes the finger together with the finger cover, with the fingertip first, into the application opening, the finger cover is efficiently formed around the finger.

In one embodiment, the application opening may have a flexible circumferential flange. To facilitate the forming of the finger cover to the finger, a flange around the circumference of the application opening may be flexible, making the diameter of the application opening flexible. The flange may be made of a flexible material such as rubber or plastic, and/or have a shape that provide a flexible diameter of the opening. The flange may for instance be formed having a plurality of slits forming a plurality of flange portions distributed along the circumference of the flange.

In one embodiment, the flexible circumferential flange may be a flange at a longitudinal end of a first tube, wherein the first tube may have a slit extending along the first tube's longitudinal length. Such tube arranged in the application opening may further provide a flexibility to the application opening, facilitating the finger cover forming to the finger. The first tube may be made of a plastic material. The first tube may provide a control of the radial flexibility of the application opening, throughout the depth of the application opening.

In one embodiment, the application opening may comprise a second tube arranged radially outside and enclosing the first tube, wherein the second tube may comprise a slit extending along the second tube's longitudinal length. The second tube may be made of a plastic material. The second tube may provide a further control of the radial flexibility of the application opening.

In one embodiment, the slit of the second tube may be arranged circumferentially separate from the slit of the first tube. In a further embodiment, the slit of the second tube may be arranged substantially circumferentially opposite to the slit of the first tube.

In one embodiment, the application opening may have a resilient bottom. The application opening may have a depth that may be varying by the resilient bottom. The bottom of the application opening may be configured to move by means of the user putting the finger and finger cover into the application opening. The bottom of the application opening may in one embodiment be spring biased. When the user puts the finger and finger cover into the application opening, the spring is compressed, and the bottom of the application opening is moved such that the depth of the application opening is increased.

In one embodiment, the application opening may be provided in an application module removeable from the housing. The application opening, which may have e.g. a flexible flange and a spring biased bottom, may be provided in a removeable application module, which may be removed e.g. for cleaning purposes or replacement purposes.

In one embodiment, the feeder mechanism may be configured to feed disposable paper finger covers. Such disposable finger covers may have a flat shape. Further, in one embodiment, the disposable finger covers may each comprise a first circular portion and a second protruding portion. The second protruding portion may e.g. be tapering or rectangular.

In one embodiment, the housing may comprise a cover reception opening which may have a shape substantially corresponding to the shape of the finger covers. The housing may have a reception opening through which the feeder mechanism may be configured to supply finger covers. In one embodiment, the reception opening may have a shape substantially corresponding to the shape of the finger covers. In case of flat finger covers, the reception opening may have a shape corresponding to the overall shape of such finger covers. In another embodiment, the reception opening may be formed as a slot configured to receive the thickness of a finger cover.

In one embodiment, the feeder mechanism may be configured to comprise a stack of finger covers fed towards the housing. The stack of finger covers may comprise a plurality of flat finger covers stacked on top of each other. The stack may extend in a longitudinal direction, and the feeder mechanism may be configured to feed the finger covers to the housing in the longitudinal direction. The housing may comprise a reception opening through which the feeder mechanism may be configured to feed finger covers from the stack of finger covers. The reception opening may have a shape corresponding to the shape of the stacked finger covers.

In one embodiment, the feeder mechanism may be configured to spring bias the stack of finger covers towards the housing. The finger covers may be spring biased in a longitudinal direction of the stack, towards the housing. The housing may comprise a reception opening, and the feeder mechanism may be configured to spring bias the stack of finger covers towards the reception opening. The feeder mechanism may comprise a spring configured to provide a spring force to the stack of finger covers in a direction towards the housing.

In one embodiment, the housing and/or the feeder mechanism may comprise a cover stop arranged above the reception opening and extending over a portion of the reception opening. The feeder mechanism may be configured to spring bias the stack of finger covers towards the reception opening and the cover stop. The cover stop may be configured to hold the stack of finger covers against the spring bias. The position of the topmost finger cover relative to the housing may thereby be controlled.

In one embodiment, the cover stop may comprise a steering pin configured to extend through a hole in the top finger covers in the stack of finger covers. The steering pin may extend in the same axial direction as the stack of finger covers. The steering pin may have a length corresponding to the thickness of 2-20 finger covers. All finger covers may comprise a hole configured to receive the steering pin. The hole and the location thereof on the finger cover may be configured to enable the finger cover to be ripped off from the steering pin when moved from the first position to the application position.

In one embodiment the feeder mechanism may be configured to comprise a roll of consecutive finger covers and to continuously supply finger covers from said roll to the housing. Instead of a stack of finger covers, the feeder mechanism may comprise a roll of finger covers. The roll of finger covers may comprise a formed string of finger covers, formed of a common roll of material, such as a paper roll. The roll may comprise a high number of consecutive finger covers, such as hundreds or thousands of finger covers. When a finger cover is moved from the first position, at which it is accessible to the user, the next finger cover may be fed from the roll. Such movement may be when the finger cover is moved from the first position to an application position, at which the finger cover may be pushed into the application opening, the application position being different from the first position, or it may be when the finger cover is pushed into the application opening, in an embodiment where the first position and the application position are the same. The consecutive finger covers may be attached to each other. Between two consecutive finger covers there may be a separation means, such as a perforation or partial opening, configured to facilitate separation of the two finger covers. The roll of finger covers may be configured for a mechanical feeding of finger covers to the housing. Alternatively, the feeder mechanism may comprise electrical driving means, such as an electrical motor, to electrically feed finger covers to the housing. The electrical driving means may be combined with one or more sensors used for activation of the electrical feeding of finger covers. Such sensor for activation or deactivation of the electrical feeding of finger covers may for instance detect the perforation or partial opening provided between two consecutive finger covers.

In one embodiment, the housing and/or the feeder mechanism may comprise a cover separation function for separation of two consecutive finger covers. The cover separation function may be activated by means of the user putting a finger with finger cover into the application opening. The cover separation function may be configured to hold or in other way prevent the subsequent finger cover to move from the first position. In an embodiment wherein the feeder mechanism comprises an electrical feeding function of finger covers, the cover separation function may be provided by the electrical feeding function holding a subsequent finger cover, causing a present finger cover to be separated from the subsequent finger cover when the present finger cover is moved from the first position or the position it was located in when the cover separation function was activated. After such separation, the electrical feeding function may be configured to feed the subsequent finger cover to the first position.

In one embodiment, the housing may comprise a separation module configured to facilitate the separation between a present finger cover to be applied to a finger and a subsequent finger cover. The separation module may comprise a first part at which a finger cover is in its first position and a second part at which the finger cover is in its application position. The second part may be pivotably attached to the first part between a resting position and a pressed position. The second part may be spring biased towards the resting position. The separation module may comprise a separation mechanism that is activated upon the second part being pivoted to the pressed position. The second part may be configured to be pivoted to the pressed position when a user puts a finger with a finger cover into the application opening. The application opening may be arranged on the second part. The mechanism may be provided as a separation projection projecting at a surface of the first part when the second part is pivoted to the pressed position. The separation projection may be configured to project between two consecutive finger covers, e.g. at an interface between such two finger covers. Such interface may comprise a separation means, such as an opening or a perforation. The projection of the separation projection into the separation means may cause or facilitate the separation of the two finger covers. Alternatively, or additionally, the mechanism may provide that when a present finger cover is located at the application opening, a subsequent finger cover at the first position is fixed at the first position. Such fixation may for instance be by means of a clamping of the subsequent finger cover between two elements, such as two elements forming a slot that may be closed in a clamping state, thereby fixing the position of the subsequent finger cover.

In one embodiment, the feeder mechanism may comprise a replaceable cartridge of finger covers to be supplied to the housing. The replaceable cartridge may comprise a stack of finger covers, or a roll of finger covers. In another embodiment, the dispenser comprises two or more rolls or stacks of finger covers. The two or more rolls or stacks of finger covers may be provided to enable additional supply of finger covers without the need of replacement of a cartridge.

According to a second aspect of the invention, a method of applying a finger cover to a user's finger is provided. The method comprises the steps of providing a present finger cover at a first position in a finger cover dispenser by means of a feeder mechanism in the finger cover dispenser, the present finger cover being a finger cover accessible to a user out of a plurality of finger covers in the dispenser; providing the present finger cover at an application position at which the present finger cover is located at an application opening of the finger cover dispenser; and pushing the present finger cover into the application opening using the user's finger such that the present finger cover is formed to enclose the user's finger. In one embodiment, a movement of the present finger cover from the first position may cause a feeding of a subsequent finger cover to the first position. Such movement may be from the first position to the application position or the pushing of the present finger cover into the application opening. The dispenser may in one embodiment be a dispenser according to any of the embodiments presented above.

In one embodiment, the first position and the application position may be different position, such that movement of the present finger cover from the first position to the application position causes a subsequent finger cover to be fed to the first position.

In one embodiment, the first position and the application position may be one and the same position, such that when the present finger cover is fed to be accessible to a user, the present finger cover is located at the application opening and enabled to be pushed therein to form the present finger cover to enclose the user's finger. The present finger cover may thereby be moved from the first position when being pushed into the application opening. When the present finger cover is pushed into the application opening, this may cause a subsequent finger cover in the finger cover dispenser to be fed to the first position by the feeder mechanism.

### Brief Description of the Drawings

The invention will in the following be described in more detail with reference to the enclosed drawings, wherein:
Fig. 1 shows a perspective view of a dispenser according to an embodiment of the present invention;
Fig. 2 shows a perspective view of a dispenser according to an embodiment of the present invention;
Fig. 3 shows a perspective view of a dispenser according to an embodiment of the present invention;
Fig. 4 shows a perspective view of a finger cover cartridge according to an embodiment of the present invention;
Fig. 5 shows a perspective view of a dispenser according to an embodiment of the present invention;
Fig. 6 shows a perspective view of a dispenser according to an embodiment of the present invention;
Fig. 7 shows a perspective view of a finger cover cartridge according to an embodiment of the present invention;
Fig. 8 shows a perspective view of a dispenser according to an embodiment of the present invention;
Fig. 9 shows a perspective view of a dispenser according to an embodiment of the present invention;
Fig. 10 shows a perspective view of an application module according to an embodiment of the present invention;
Fig. 11 shows a perspective view of an application module according to an embodiment of the present invention;
Fig. 12 shows a perspective view of a separation module according to an embodiment of the present invention;
Fig. 13 shows a perspective view of a separation module according to an embodiment of the present invention;
Fig. 14 shows a perspective view of a separation module according to an embodiment of the present invention;
Fig. 15 shows a perspective view of a separation module according to an embodiment of the present invention;
Fig. 16 shows a perspective view of a user's finger applying a finger cover according to an embodiment of the present invention; and
Fig. 17 shows a perspective view of a user's finger with an applied finger cover according to an embodiment of the present invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

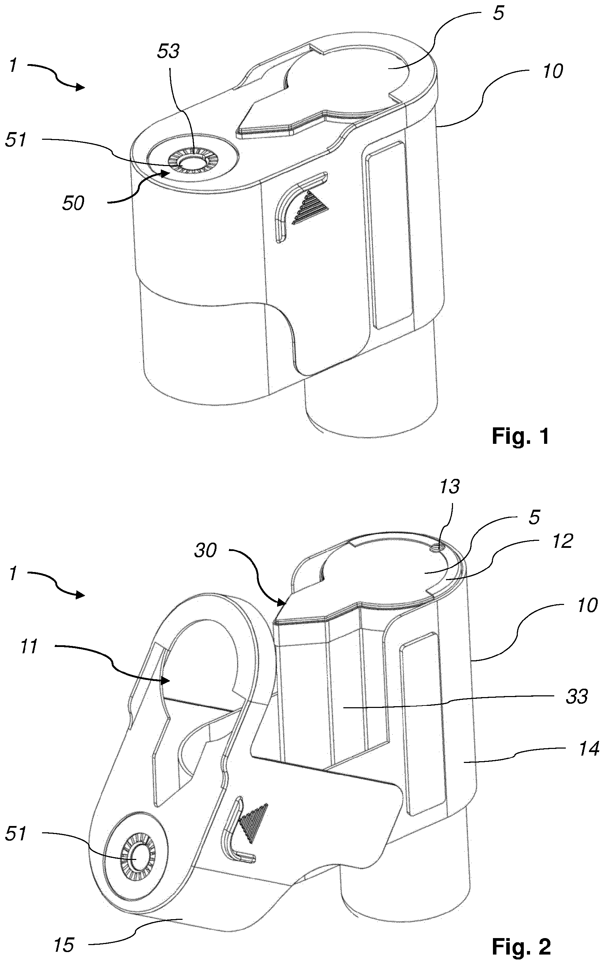

Figs. 1 and 2 illustrate a finger cover dispenser 1 according to an embodiment of the invention. The dispenser 1 comprises a housing 10 and a feeder mechanism 30. The housing 10 encloses the feeder mechanism 30. The feeder mechanism 30 is configured to feed finger covers 5 to be accessible for a user. The housing comprises a reception opening 11 for receiving finger cover 5, thereby making it accessible to a user.

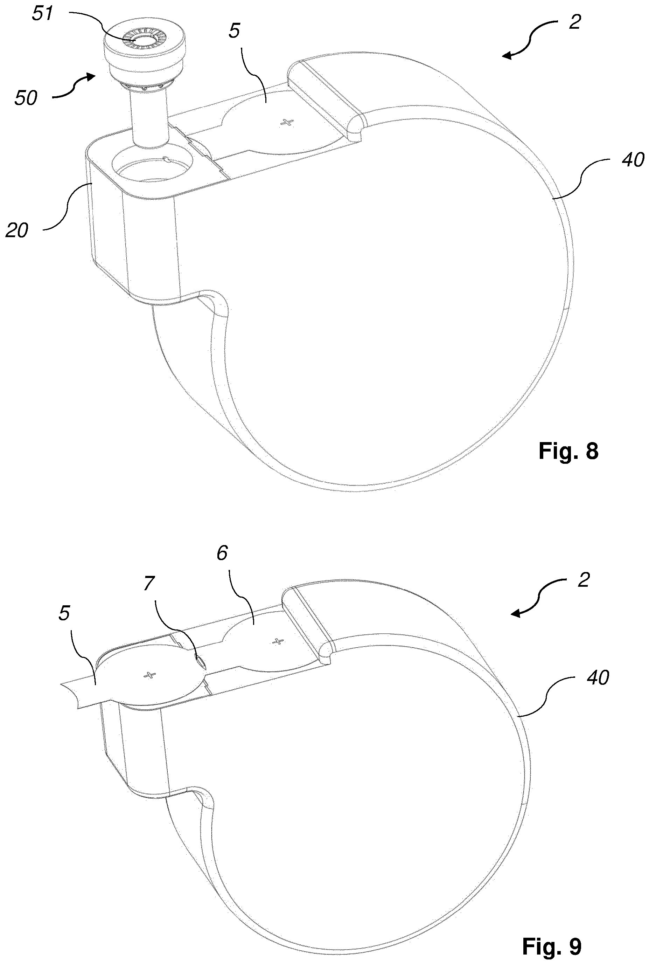

The housing 10 comprises an application opening 51. The application opening 51 is part of an application module 50 provided in the housing 10 (see further in figure 9). The application opening 51 is configured to receive a user's finger together with a finger cover 5. When the user puts the finger and the finger cover 5 into the application opening 51, the finger cover 5 is formed to be applied to the user's finger.

The housing 10 comprises a first part 14 and a second part 15. The housing 10 is openable by movement of the second part 15 relative to the first part 14. In the illustrated embodiment, such movement is a pivotal movement, rotating the second part 15 relative to the first part 14. By opening the housing 10, the feeder mechanism 30 may be accessed. The feeder mechanism 30 may be accessed in order to refill the feeder mechanism 30 with new finger covers 5.

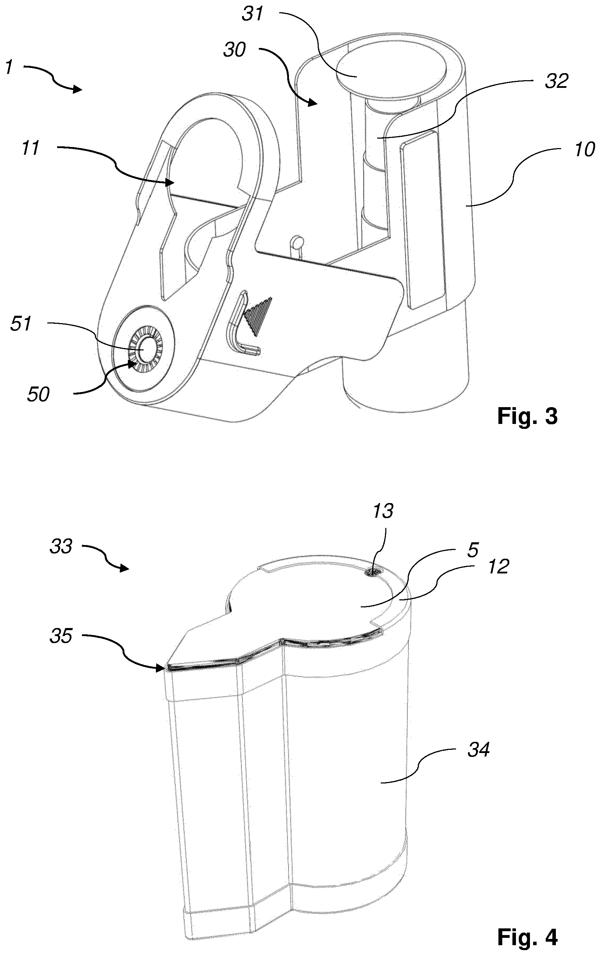

Fig. 3 illustrates the dispenser 1 with an opened housing 10 and without any finger covers 5 supplied in the feeder mechanism 30. The finger covers 5 is provided in the feeder mechanism 30 in a cartridge 33, as illustrated in fig. 4. The cartridge 33 comprises a cartridge enclosure 34 enclosing a stack 35 of finger covers 5. The feeder mechanism 30 further comprises a bottom plate 31 being spring biased by means of a spring function 32. When the cartridge 33 is mounted in the feeder mechanism 30 within the housing 10, the spring biased bottom plate 31 is configured to push on the stack 35 of finger covers 5. Finger covers 5 from the stack 35 are thereby supplied at the top of the feeder mechanism 30. A finger cover 5 at the top is thereby accessible to a user.

The housing 10 and/or the feeder mechanism 30 may comprise a cover stop 12 configured to hold the stack 35 of finger covers 5 being spring biased towards the housing 10. The spring force of the spring function 32 thereby acts towards the cover stop 12. The cover stop 12 extends over a portion of the finger cover 5 at the top of the stack 35. The cover stop 12 may be provided in connection to the cartridge enclosure 34, as seen in figs. 2 and 4. Alternatively or additionally, the cover stop may be formed in the housing as seen in fig. 1.

The cartridge 33 and/or the housing 10 may further comprise a steering pin 13 configured to extend towards and through the stack 35 of finger covers 5. All finger covers 5 of the stack 35 may comprise a hole 8 for receiving a steering pin 13. The steering pin 13 preferably extends through the topmost 2 to 20 finger covers 5 of the stack 35. The steering pin 13 may be formed as part of the cartridge 33 as illustrated in fig. 4. Alternatively, the steering pin may be formed as part of the housing 10.

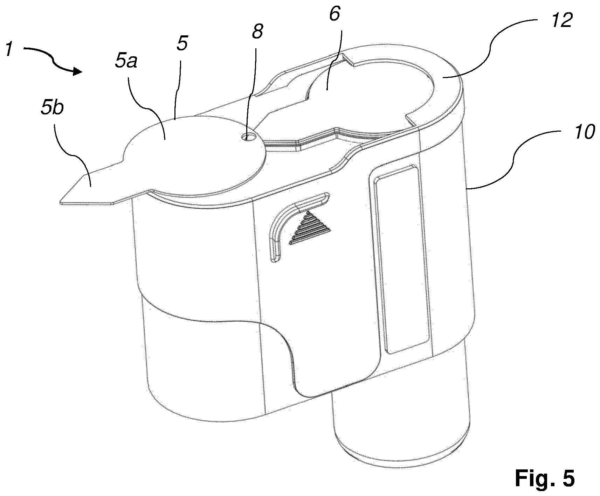

Fig. 5 illustrates when the present, topmost, finger cover 5 has been moved from the first position to the application position. When the present finger cover 5 has been moved to the application position, a subsequent finger cover 6 is fed by the feeder mechanism to the first position. In the first position, the subsequent finger cover 6 thereby becomes accessible for subsequent application to a user's finger. When in the application position, the finger cover 5 is located above the application opening 51, enabling a user to put the finger into the application opening 51 together with the finger cover 5, thereby applying the finger cover 5 to the finger. In one embodiment, the finger cover 5 comprises a first circular portion 5a and a second protruding portion 5b extending from said first circular portion 5a. When the finger cover 5 is applied to the user's finger, formed to enclose the finger, the second protruding portion 5b may facilitate removal of the finger cover 5 from the finger to which the finger cover 5 was applied. The removal may be performed by moving another finger in between the second protruding portion 5b and the finger to which the finger cover 5 is applied.

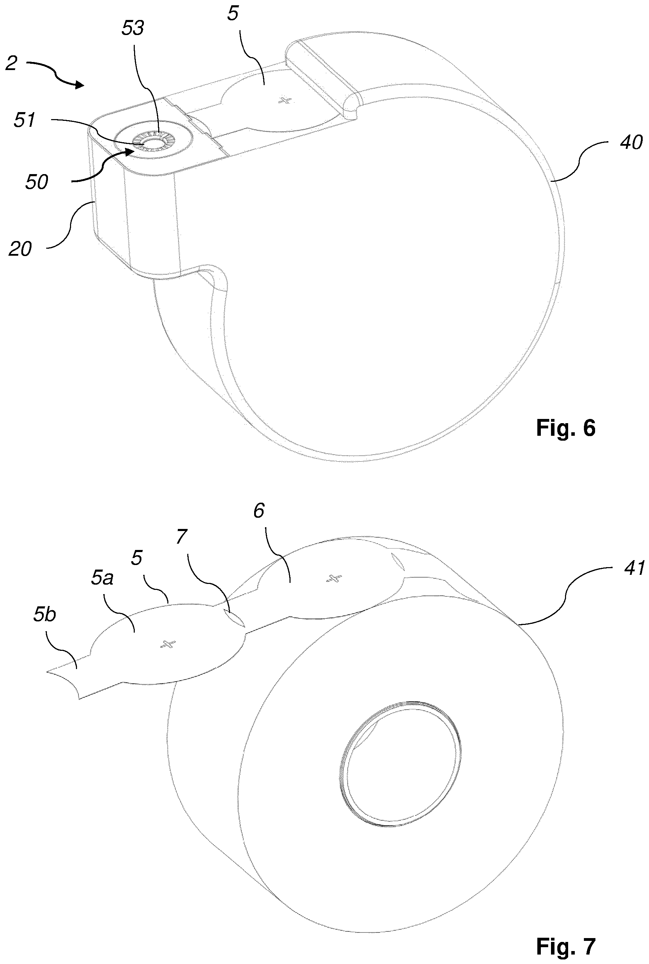

Figs. 6-9 illustrates a second embodiment of the invention, comprising a dispenser 2 with a housing 20 and a feeder mechanism 40. The housing 20 comprises an application opening similar to the dispenser 1 of the first embodiment. The dispenser 2 further comprises a feeder mechanism 40 configured to feed a present finger cover 5 to be accessible at a first position to a user.

As seen in fig. 7, the feeder mechanism 40 comprises a roll 41 of finger covers 5, 6. The present finger cover 5 is feed to be accessible at the first position to a user as illustrated in fig. 6. A subsequent finger cover 6 follows the present finger cover 5 in the roll 41. The roll 41 is configured to rotate to feed finger covers 5, 6 to be accessible to a user. Between the finger covers 5, 6 of the roll 41 there is provided a separation means 7. The separation means may be provided as transverse opening, a perforation or the like. In the illustrated embodiment, the separation means 7 is provided as a transverse opening extending along a portion of width of the interface between the two finger covers 5, 6, centered in the transverse direction. The finger covers 5, 6 each comprises a first circular portion 5a and a second protruding portion 5b. The separation means 7 is provided between the first circular portion 5a of one finger cover 5 and the protruding portion 5b of a subsequent finger cover 6.

As illustrated in fig. 8, the housing 20 comprises an application module 50 providing the application opening 51. The application module 50 is removeable and exchangeable.

Fig. 9 illustrates the dispenser 2 when the present finger cover 5 has been moved from the first position to the application position. When a user moves the present finger cover 5 from the first position to the application position, the subsequent finger cover 6 is fed to the first position from the roll 41. In the first position, the subsequent finger cover 6 thereby becomes accessible for subsequent application to a user's finger. When in the application position, the present finger cover 5 is located above the application opening 51, enabling a user to put the finger into the application opening 51 together with the finger cover 5, thereby applying the finger cover 5 to the finger. When the finger cover 5 is applied to the user's finger, formed to enclose the finger, the second protruding portion 5b may facilitate removal of the finger cover 5 from the finger to which the finger cover 5 was applied. The removal may be performed by moving another finger in between the second protruding portion 5b and the finger to which the finger cover 5 is applied.

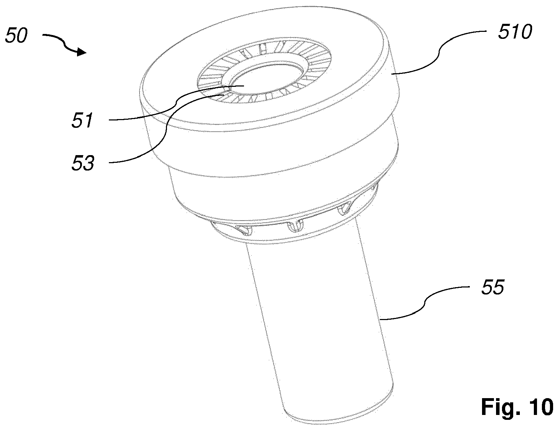

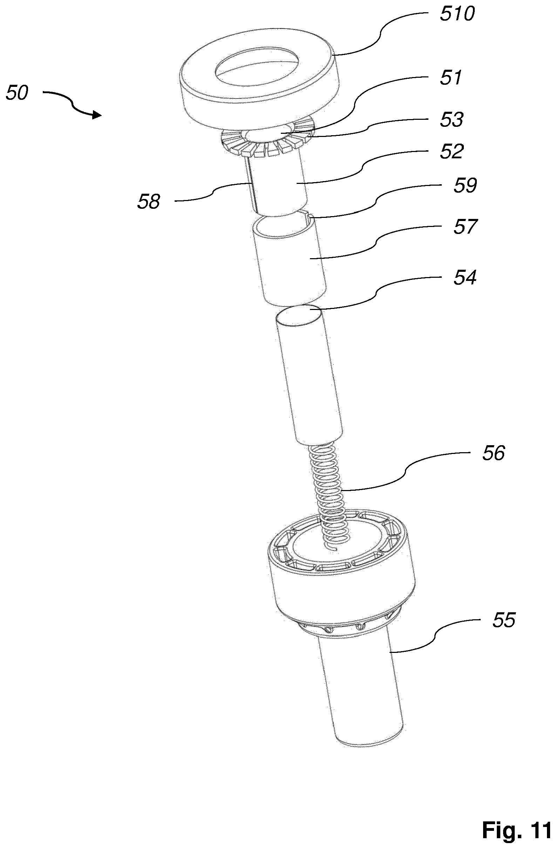

Figs. 10-11 illustrates the application module 50 comprising the application opening 51. The application module 50 comprises a base part 55. In the application module 50 a bottom member 54 is provided, constituting the bottom of the application opening 51. The bottom member 54 is arranged on a spring member 56, biasing the bottom member 54 towards the top of the application module 50. The depth of the application opening 51 is thereby variable. When the user puts the finger with the finger cover 5 into the application opening 51, the finger touches the bottom member 54, and when the finger is further pushed into the application opening 51 to completely apply the finger cover 5 to the finger, the finger pushes the bottom member 54 further down, compressing the spring 56. The resilient bottom of the application opening 51 facilitates the application of the finger cover 5 to the finger.

The application module 50 further comprises a first cylindrical tube member 52 and a second cylindrical tube member 57. The second cylindrical tube member 57 is arranged to circumferentially enclose the first cylindrical tube member 52. The two tube members 52, 57 are arranged to enclose the bottom member 54.

Each of the two tube members 52, 57 comprises a longitudinal slit 58, 59 extending along the axial extension of each of the tube members 52, 57. The slits 58, 59 makes each of the tube members 52, 57 radially expandable. The slit 58 of the first cylindrical tube member 52 is arranged circumferentially opposite to the slit 59 of the second cylindrical tube member 57.

At the longitudinal end of the first cylindrical tube member 52 facing towards the application opening 51, a circumferential flexible flange 53 is provided. The flange 53 extends in transverse direction to the longitudinal extension of the first cylindrical tube member 52. The flange 53 comprises a plurality of flange portions, together forming the flexible flange 53.

The application module further comprises a cap member 510 configured to be attached to the base part 55. The application module 50 may be removeable from the housing 10, 20 and/or openable such that it may be replaced and/or cleaned.

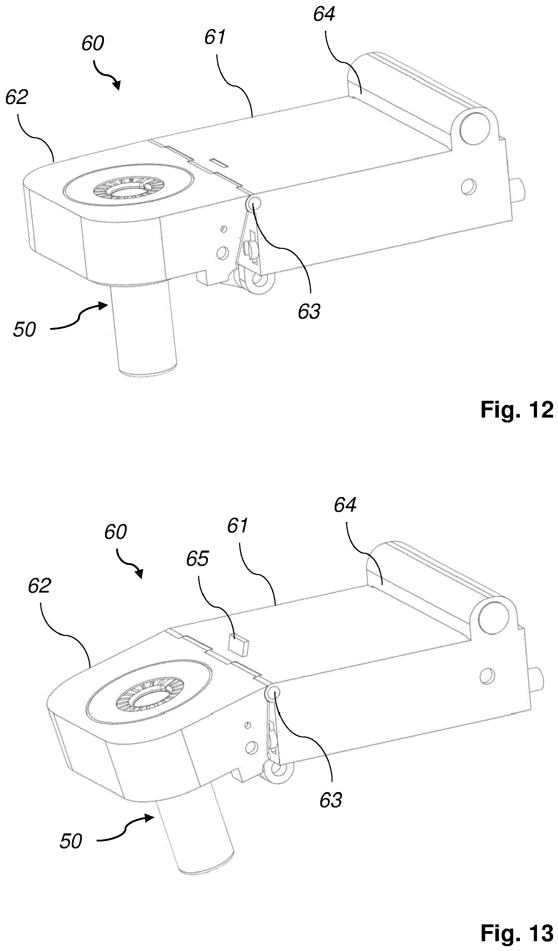

Figs. 12-13 illustrates an embodiment of a separation module 60 that may be part of the dispenser 2. The separation module 60 comprises a first part 61 that is fixed to the housing 20, and a second part 62 that is pivotally suspended on the first part 61 around a pivot point 63. The separation module 60 further comprises a slot 64 through which finger covers 5, 6 is fed from the feeder mechanism 40. Fig. 12 illustrates the second part 62 in a resting position. The second part 62 is spring biased towards the resting position.

Further, the separation module 60 comprises a separation projection 65 that extends on the first part 61 when the second part 62 is pivoted to a pressed position as illustrated in fig. 13. The separation module 60 comprises a mechanism that projects the separation projection 65 when the second part 62 is pivoted from the resting position towards the pressed position. The separation projection 65 projects into the separation means 7 between two consecutive finger covers 5, 6 when the present finger cover 5 has been moved to the application position.

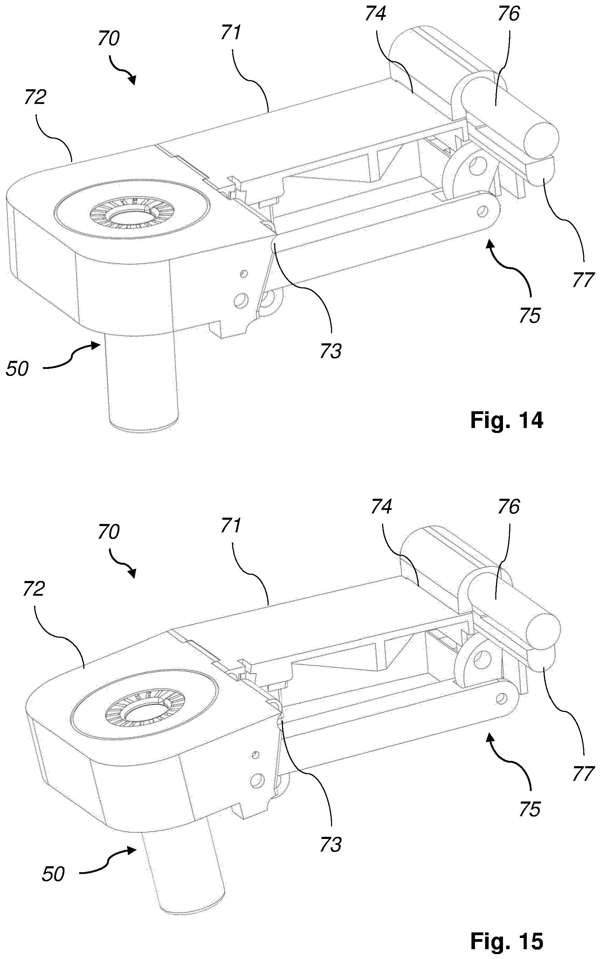

Figs. 14-15 illustrates an embodiment of a separation module 70 which may be combined with the features of the separation module 60 illustrated in figs. 12-13. The separation module 70 comprises a first part 71 that is fixed to the housing 20, and a second part 72 that is pivotally suspended on the first part 71 around a pivot point 73. The separation module 60 further comprises a slot 74 through which finger covers 5, 6 is fed from the feeder mechanism 40. Fig. 14 illustrates the second part 62 in a resting position. The second part 72 is spring biased towards the resting position.

The slot 74 is provided by two slot elements 76, 77 which in the resting position of the second part 72 is arranged with a distance between each other. In a pressed position, wherein the second part 72 is pivoted relative to the first part 71, a mechanism 75 provides that the two slot elements 76, 77 are pressed towards each other. When a finger cover 5, 6 is present in the slot 74 is thereby held by the slot elements 76, 77 when the second part 72 is pressed. The subsequent finger cover 6 may thereby be held in the first position by the slot 74 when a present finger cover 5 is put into the application opening 51 to be applied to a user's finger.

When a present finger cover 5 is in the application position and a user puts a finger together with the finger cover 5 into the application opening 51 of the application module 50, the second part 62, 72 of either of the embodiments of the separation module 60, 70, is pivoted relative to the first part 61, 71 which activates the mechanism of the separation projection 65 and/or slot elements 76, 77 to facilitate the separation of the present finger cover 5 and the subsequent finger cover 6.

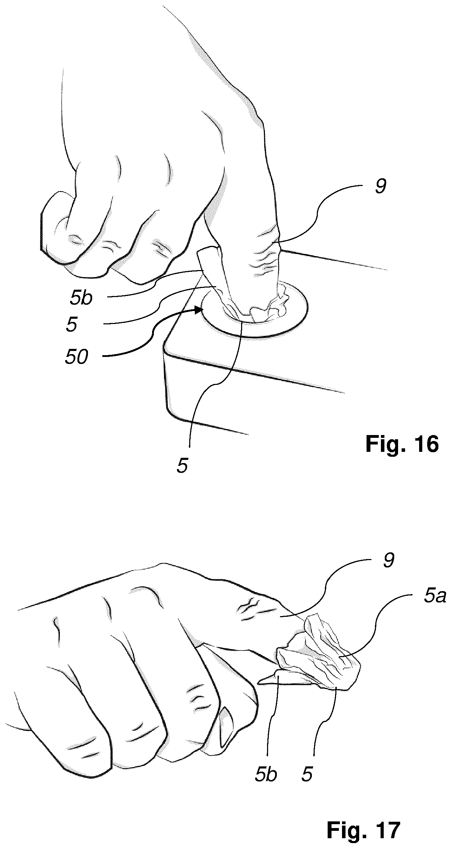

Figs. 16 and 17 illustrates a user's finger 9 pushing the finger cover 5 into the application opening 51 in the application module 50. The illustrated application of the finger cover 5 is associated with either of the embodiments of the finger cover dispenser 1, 2 as presented herein. In fig. 16 the finger cover 5 located at the application position above the application opening 51 is pushed into the application opening 51 by the user's finger 9. The cylindrical hole of the application opening 51 forms the finger cover 5 to enclose the user's finger 9, especially the fingertip. The user's finger 9, with the finger cover 5, hits the bottom member 54 of the application module 50. When the finger 9 is further pushed into the application opening 51, the spring biased bottom member 54 is pushed down, which helps the finger cover 5 to be formed correctly around the user's finger 9. The tube members 52, 57 of the application module 50 may further facilitate the forming of the finger cover 5 around the user's finger 9. The flexibility of the tube members 52, 57 helps in providing a suitable forming of the finger cover 5 also for different sizes of fingers.

As seen in fig. 17, the user's finger 9 has been removed from the application opening 51 after application of the finger cover 5 therein. The finger cover 5 is formed around the user's finger 9 and may be used as a finger protection on e.g. touch screens.

After application of the finger cover 5 on the user's finger 9, the first circular portion 5a of the finger cover 5 is the part of the finger cover 5 that mainly encloses the fingertip of the user's finger 9. The second protruding portion 5b extends at e.g. an underside of the finger 9. The second protruding portion 5b may be used when removing the finger cover 5 from the finger 9 by sticking another finger between the second protruding portion 5b and the finger 9, and moving the other finger towards the fingertip of the finger 9.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A finger cover dispenser (1, 2) comprising
a housing (10, 20), and
a feeder mechanism (30, 40) configured to feed finger covers (2) to the housing,
wherein the housing comprises
an application opening (50) which is configured to, when in use, enable a user to apply the finger cover (5) to a finger (9) by putting the finger together with a finger cover received from the feeder mechanism into the application opening.

2. The finger cover dispenser according to claim 1, wherein the application opening (50) is a circular opening having a diameter of between 10-30 mm, preferably between 12-20 mm.

3. The finger cover dispenser according to any of claims 1 and 2, wherein the application opening (50) has a flexible circumferential flange (52).

4. The finger cover dispenser according to claim 3, wherein the flexible circumferential flange (52) is a flange at a longitudinal end of a first tube (51), wherein the first tube has a slit (58) extending along the first tube's longitudinal length.

5. The finger cover dispenser according to any of the preceding claims, wherein the bottom (53) of the application opening (50) is spring biased.

6. The finger cover dispenser according to any of the preceding claims, wherein the application opening (51) is provided in an application module (50) removeable from the housing (10, 20).

7. The finger cover dispenser according to any of the preceding claims, wherein the feeder mechanism (30, 40) is configured to feed disposable finger covers (5) having a flat shape.

8. The finger cover dispenser according to any of the preceding claims, wherein the feeder mechanism (30, 40) is configured to feed disposable finger covers (5) comprising a first circular portion (5a) and a second protruding portion (5b).

9. The finger cover dispenser according to any of the preceding claims, wherein the feeder mechanism (30) comprises a stack (35) of finger covers to be fed to the housing (10).

10. The finger cover dispenser according to claim 9, wherein the housing (10) comprises a reception opening (11) though which the feeder mechanism (30) is configured to feed finger covers (5), wherein the reception opening (11) has a shape corresponding to the shape of the stacked finger covers.

11. The finger cover dispenser according to any of the claims 9-10, wherein the dispenser (1) comprises a cover stop (12) arranged above the reception opening (11) and extending over a portion of the reception opening.

12. The finger cover dispenser according to claim 11, wherein the cover stop (12) comprises a steering pin (13) configured to extend through a hole in the top finger covers (5) in the stack of finger covers.

13. The finger cover dispenser according to any of the claims 1-8, wherein the feeder mechanism (40) is configured to comprise a roll (41) of consecutive finger covers (5) and to feed finger covers from said roll to the housing (20).

14. The finger cover dispenser according to claim 13, wherein the housing (20) and/or the feeder mechanism (40) comprises a cover separation function for separation of two consecutive finger covers (5).

15. A method of applying a finger cover to a user's finger, the method comprising the steps of:
providing a present finger cover (5) at a first position in a finger cover dispenser (1, 2) by means of a feeder mechanism in the finger cover dispenser, the present finger cover being a finger cover accessible to a user out of a plurality of finger covers in the dispenser;
providing the present finger cover (5) at an application position at which the present finger cover is located at an application opening (51) of the finger cover dispenser (1, 2); and
pushing the present finger cover (5) into the application opening (51) using the user's finger (9) such that the present finger cover is formed to enclose the user's finger.
